# EUROPEAN PATENT SPECIFICATION

(11) **EP 2 295 098 B1**
(45) Date of publication and mention of the grant of the patent: **26.10.2011**
(21) Application number: 09169936.3
(22) Date of filing: 10.09.2009
(51) Int. Cl.: A61M 5/172, A61M 5/142

(54) **Medical infusion pump and method for determining the cause of a power supply interruption in the pump**
Medizinische Infusionspumpe und Verfahren zur Bestimmung der Ursache einer Stromunterbrechung in der Pumpe
Pompe à perfusion médicale et procédé pour déterminer la cause d'interruption d'alimentation électrique de la pompe

(43) Date of publication of application: 16.03.2011
(73) Proprietor: F. Hoffmann-La Roche AG, 4070 Basel (CH); Roche Diagnostics GmbH, 68305 Mannheim (DE)
(72) Inventor: Rufer, Thomas, 3072 Ostermundigen (CH); Aeschlimann, Reto, 3426 Aefligen (CH)
(74) Representative: Schwabe - Sandmair - Marx

(56) References cited:
- US-A- 5 321 392
- US-A- 5 764 034
- US-A- 5 876 423
- US-A1- 2002 065 454
- US-A1- 2007 040 449
- US-A1- 2007 293 914

## Description

The present invention relates to a medical infusion pump, in particular to an ambulatory medical infusion pump, and a method for determining the cause of an interruption of a power supply provided by a power source in the medical infusion pump. Such infusion pumps can be, for example, insulin pumps, in particular worn by a person continuously night and day.

Medical devices like medical infusion pumps are typically powered by a power source. A failure occurs if the voltage provided to the medical device or specific components within the medical device drops to or below a critical value, which is also called a reset voltage level because a reset of the medical device or a specific component within the medical device is performed at this voltage level. In particular for liability reasons, it is important to know if the reason for the interruption of the power supply was a misuse of the device by the user, for example like removing the power source or dropping the device, or if the reason lies within the medical device, in particular within the power source, itself.

Document US 5,764,034 discloses a battery gauge for a battery operated infusion pump. In this document, formulas are given for calculating the time left to any remaining battery voltage based on six stored voltage values, each of which represents the overage of 10 voltage samples taken at six-second intervals.

Document US 2007/0293914 A1 discloses a system and method for detecting the status of a recharchable battery included within an implantable medical device. The battery voltage is measured at a predescribed time interval, for example once every 4 hours. Should the battery voltage fall below a minimum level, the battery voltage is then measured once every 10 minutes.

It is therefore advantageous to provide a medical infusion pump and a method for at least setting the stage for determining the cause of an interruption of a power supply provided by a power source in the medical infusion pump.

Such a medical infusion pump is claimed in claim 1 and such a method is claimed in claim 10. Specific embodiments are described in the dependent claims.

A medical infusion pump according to the present invention comprises a power source, a processor and a voltage supervisor. The voltage supervisor is configured to monitor the input voltage of the processor and the voltage supervisor is further configured to respond if the voltage drops to a predetermined value which has a level which is above the reset voltage level of the processor. The predetermined value may be adaptive and be selected, for example, in dependence of the battery type, the temperature, and further factors.

In this document, "responding" of the supervisor means that the supervisor provides an output signal which indicates that the voltage has dropped to a specific level. Such a supervisor continuously compares the input voltage with an internal reference level. The reset voltage level is a voltage level at which the processor performs a reset. After the reset, the processor is powered up. The reset voltage level is typically, but not necessarily slightly higher than the minimum voltage level required for operation of the processor. The voltage supervisor may be external to the processor, an internal component of the processor or a functionality of the processor, for example using an internal A/D converter and a logic.

The processor is configured to start logging the voltage of the power source, thus creating a profile of the voltage over time, when the voltage supervisor responds. The logging is carried out with a sufficiently high sampling rate to ensure that a number of values is logged before the voltage drops to the reset voltage level. It is typically in the range of some kHz.

The logged data can be analyzed using an external device which is configured to receive the logged data from the medical infusion pump to determine the cause of an interruption of the power supply to the processor from the logged power source voltage. In another embodiment, the processor is further configured to determine the cause of an interruption of the power supply to the processor from the logged power source voltage. If the power source is removed, the voltage of the power source rapidly falls to zero or near zero and remains there. The voltage may fall to near zero instead of zero if there is a capacitor present parallel to the battery. The residual voltage level, which falls under the term "near zero", can have levels of up to 0.5 volts, for example. If the medical device is dropped, such that the contacts, like a contact spring, temporarily detach, there is a steep notch in the power source voltage to zero or near zero for a short period of time. These two events indicate a misuse of the device by the user. At the time the input voltage of the processor reaches the reset voltage level, the power source voltage is either still zero (or close to zero) in the case of a removed battery or may have recovered in the case of a dropped device.

In another event in which the battery is weak, the drop in the power source voltage is not as steep and not as deep as when the power source is removed or temporarily disconnected. This means that the profile of the power source voltage is different. In particular, at the time when the processor input voltage reaches the reset voltage level, the power source voltage is higher than in the first two events. Therefore, the cause of the interruption of the power supply to the processor can be determined from the logged power source voltage profile, in particular from the voltage value or level at the time when the processor input voltage reaches the reset voltage level.

One possibility for determining the cause of the power supply interruption is to compare the logged power source voltage to predetermined reference power source voltage profiles representing the cause of the power source voltage for the different events over time.

The voltage at which the supervisor responds is above the reset voltage level of the processor. This has the advantage that there is still a time span between responding of the voltage supervisor and the input voltage of the processor reaching the reset voltage level. In this time span, the processor is able to log the voltage of the power source. In general, the voltage at which the voltage supervisor responds is below the minimum voltage level which may occur during normal operation of the device due to general variation, temperature effects, etc, but above the reset voltage level of the processor. In particular, the voltage at which the supervisor responds may be 0.1 volts above the reset voltage level of the processor.

The processor can be any central processing unit (CPU) or any microprocessor, microcontroller or ASIC. The battery can be a single-use battery or rechargeable battery, for example.

As an option, there is a voltage converter provided between the power source and the processor. This voltage converter converts the output voltage of the power source, which might be between 1.2 and 1.5 volts, for example, to the operating voltage of the processor, which might be 3 volts, for example. In one embodiment, the voltage converter is a DC-DC-converter. As an option, the voltage converter may contain a power buffer, such as a capacitor, for stabilizing its output voltage.

In one embodiment of the present invention, the voltage supervisor is connected to an interrupt input port of the processor. With this configuration, the processor is automatically aware that its input voltage has dropped to a predetermined level without the need to actively sample the input to which the supervisor is connected. Automatic sampling is less favorable with respect to energy consumption since the processor is typically in a low energy mode most of the time but has to be activated for each sampling.

In one embodiment of the present invention, the processor is configured to log the power source voltage until the processor is reset. The processor is reset when the processor input voltage reaches the reset voltage level. The power source voltage level profile around the time the processor input voltage reaches the reset voltage level is indicative of the event that caused the drop in the power source voltage level. In another embodiment, the processor is configured to log the power source voltage until the voltage supervisor stops responding. The voltage supervisor stops responding when the processor input voltage rises to or above the predetermined voltage level at which the voltage supervisor responds. In this case, a short-term power interruption, for example caused by dropping the medical device, can be detected even if the processor input voltage does not drop to the reset voltage level.

As an option, the processor is configured to perform alternative logging of power source voltage values to different memories. This has the advantage that the voltage levels stored in one memory are not affected when the processor is reset during storing voltage levels to the other memory. In addition, the history of voltage levels stored in each memory, which is therefore also called history memory, contains a reduced amount of data which can be sufficient for evaluating the cause of the power supply interruption. The different memories can either be different physical memories or different areas of the same physical memory.

The history memory is favourably a non-volatile memory such that the logged voltage values are still available after a succeeding power up and may, for example, be a static RAM. If the logged battery voltage drops to or below the reset voltage level, the logged profile should be stored permanently and can not be cleared by the user of the device but only by a technical expert who analyzes the device or by a service person.

In a preferred embodiment, the medical infusion pump comprises a clock circuit, which preferably continues running even if the power supply is interrupted. The clock circuit can be a part of the processor or a separate unit.

In a preferred embodiment, the processor is configured to log a timestamp together with the power source voltage. The timestamp can be retrieved from the clock circuit. Dropping the medical device, which leads to a short-term disconnection of the power source, basically has the same effect on the logged voltage as removing the power source. However, after an interruption caused by dropping the device, the power supply is interrupted for a period much shorter than when the power source is removed and re-inserted or replaced by the user. Therefore, analyzing the timestamp of the logged power source voltage values compared to the time the processor is powered up again, a drop of the device can be distinguished from removal of the power source. The time at which the processor is powered up again can also be retrieved from the clock circuit.

The present invention also relates to a method for determining the cause of an interruption of a power supply provided by a power source in a medical infusion pump, the medical infusion pump comprising a processor. The method comprises the steps of detecting if the input voltage of the processor drops to a predetermined level which is higher than the reset voltage level of the processor, starting to log the voltage of the power source when the input voltage has dropped to the predetermined voltage level and determining the cause of an interruption of the power supply from the logged power source voltage. Details of the effects of this method and its advantages as well as the predetermined voltage level, the reset voltage level and ways for determining the cause of the power supply interruption have already been explained above with reference to the medical infusion pump and are therefore omitted.

Preferably, the power source voltage is logged until the processor input voltage drops to the reset voltage level of the processor. As an addition or as an alternative, the power source voltage is logged until the processor input voltage rises to or above the predetermined voltage or to or above a threshold voltage. The threshold value might be the same as the predetermined voltage or different from the predetermined voltage, in particular higher than the predetermined voltage. The predetermined voltage level is the level at which the supervisor responds.

In an embodiment, the power source voltage values are logged alternatively to different memories. In another embodiment, a timestamp is logged together with the power source voltage.

Some or all features of different embodiments described above can be combined to new embodiments which are also covered by the present invention. In addition, the principles of the present invention can also be applied to all kinds of medical or non-medical devices with a power source, and not just to medical infusion pumps.

The present invention shall be explained in more detail with reference to a preferred exemplary embodiment and the accompanying drawings which show:
- Figure 1: a schematic block diagram of a medical infusion pump,
- Figure 2: voltage level profiles when a power source is removed,
- Figure 3: voltage level profiles for a weak battery and
- Figure 4: voltage level profiles for a dropped infusion pump.

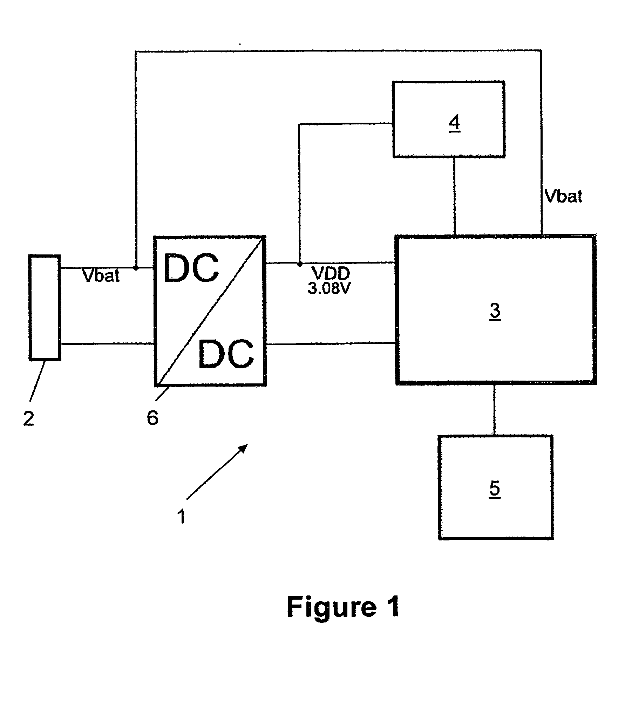

Figure 1 shows a schematic block diagram of a medical infusion pump 1, comprising a battery 2, a processor 3, a voltage supervisor 4, a memory 5 and a voltage converter 6. The battery 2 is connected to the voltage converter 6. In the present embodiment, the voltage converter 6 is a DC-DC-converter which converts the output voltage of the battery 2, which is typically between 1.2 volts and 1.5 volts, for example, to the operating voltage of the processor 3, which in the present exemplary embodiment is 3.08 volts. The output voltage of the battery 2 is called Vbat, the output voltage of the voltage converter 6, which is the input voltage of the processor 3, is called VDD. In the present embodiment, the reset voltage level of the processor 3, that is the voltage level at which the processor 3 performs a reset, is 2.6 volts.
The processor 3 controls an insulin pump (not shown) to deliver insulin from a reservoir (not shown) to a patient.

The voltage supervisor 4 is connected to the output of the voltage converter 6 such that it can monitor the voltage VDD. If the voltage VDD falls to a predetermined level, for example 2.7 volts, the voltage supervisor 4 responds and generates an output signal indicative of this event. The output of the voltage supervisor 4 is connected to an input of the processor 3, preferably to an interrupt input port of the processor 3. Another input of the processor 3 is connected to the battery 2 such that the voltage battery Vbat can be determined. In this configuration, the processor 3 comprises an A/D converter which converts the analogue voltage Vbat into a digital value which can be processed by the processor 3. As an alternative, the A/D converter can be external to the processor 3.

If the supervisor 4 indicates that the processor input voltage VDD has dropped to a predetermined voltage level, for example 2.7 volts, the processor 3 starts sampling and logging the battery output voltage Vbat and stores the values into a memory 5 which is connected to the processor 3 either directly or via a link such as a data bus.

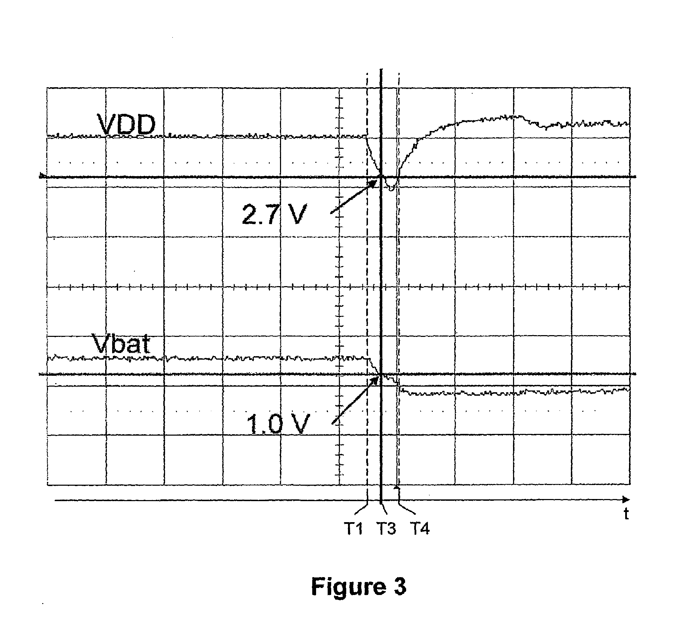

The cause for the voltage VDD dropping to a predetermined value can be one of several events. One event is that the battery 2 becomes weak. In this case, the internal resistance of the battery 2 causes a slight reduction of the battery voltage Vbat. An exemplary profile of Vbat over time is depicted in Figure 3. At a time T1, the voltage Vbat starts decreasing. About that time, the processor input voltage VDD starts falling. At the time T3, VDD has fallen to a predetermined voltage level such that the voltage supervisor 4 responds and notifies the processor 3. Accordingly, the processor 3 starts logging the battery voltage Vbat in the memory 5. In the present example, the battery output voltage Vbat falls from a nominal voltage of 1.3 volts to a reduced voltage of 0.9 volts and remains about that level.

The voltage converter 6 is able to stabilize the voltage VDD even from the reduced input voltage. As can be seen from Figure 3, the voltage converter 6 can compensate the drop in the input voltage. At the time T4, the voltage VDD rises above the predetermined voltage level of 2.7 volts. At that time, the processor 3 stops logging Vbat. From the battery voltage Vbat being 0.9 volts at the time T4, it can be determined that the cause of the drop of the processor input voltage VDD was within the battery 2, that is, the battery is of poor quality and has a high internal resistance.

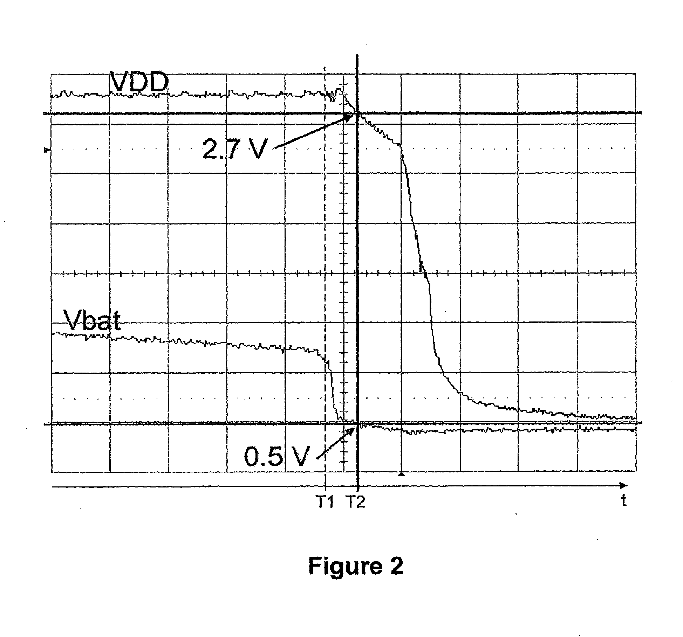

Figure 2 shows the profiles of Vbat and VDD for a second event in which the user of the infusion pump 1 removes the battery 2. In this case, when the battery 2 is removed at a time T1, the voltage Vbat rapidly drops from the nominal value of 1.3 volts to a level of 0.5 volts. The 0.5 volts level results from an internal buffer capacitor (not shown) in the power supply path. In an unbuffered system, the voltage level would be zero. About that time T1, the processor input voltage VDD starts falling. At a time T2, the voltage VDD has reached a predetermined level, 2.7 volts in the present example, such that the voltage supervisor 4 responds and notifies the processor 3. The processor 3 then starts logging the battery voltage Vbat in the memory 5. Since the voltage converter 6 is not able to stabilize its output voltage VDD, it falls beyond a level at which the processor 3 is reset. Form the logged voltage it can be seen that the battery voltage was already low at the time when the logging was started, in particular considerably lower as in the case of a weak battery discussed above with reference to Figure 3. Therefore, it can be concluded that the battery was not present at that time, i.e., that a misuse has occurred by removing the battery with the device being in operation mode. When the power supply of the device 1 is restored, the processor 3 powers up again. If the time between VDD falling to the reset voltage of the processor 3 and the time at which the processor 3 is powered up again is longer than a predetermined time span, this indicates that the battery 2 was (temporarily) removed.

This time span can be determined as follows: When the device is powered up, it may run through a self-testing and powering-up sequence of several seconds. After completing the power-up sequence, a corresponding entry is stored in the history memory of the device. If the time between the beginning of the logging, or the last logged value before VDD has fallen to the reset voltage level, and the power-up entry is considerably (i.e. in the range of half a second or more) longer than the time required for powering up, the battery had been removed. Otherwise, the battery was disconnected only for a very short period, e.g., because of a dropping of the device.

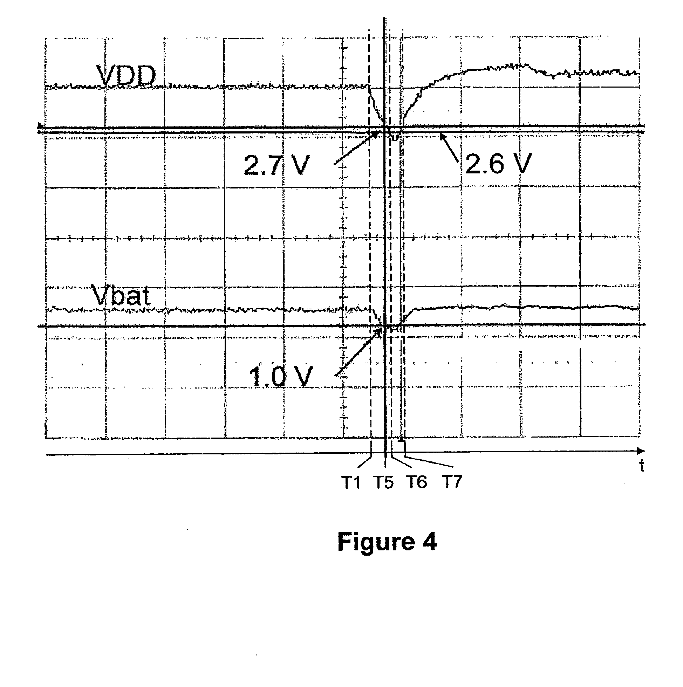

In this case of dropping the device 1, the power supply of the medical infusion pump 1 is interrupted only for a short period of time as shown in Figure 4, for example if a spring which establishes the electrical contact with an electrode of the battery temporarily disconnects. This means that the voltage Vbat sharply drops at a time T1 to the predetermined level at time T5. At time T6, the voltage VDD falls to the reset level of the processor 3 as shown in Figure 4, so that the processor 3 is reset and powered up again when VDD rises to the reset voltage again at time T7. After completing the power-up sequence, a corresponding entry is stored in the history memory 5 of the device. If the time between the beginning of the logging, or the last logged value before VDD has fallen to the reset voltage level, and the power-up entry is only slightly (i.e. in the range of milliseconds or even microseconds) longer than the time required for powering up, there was an interruption in the power supply most likely caused by a drop of the device 1.

The interruption of the power supply may be so short that VDD does fall below the predetermined voltage, but not to or below the reset voltage of the processor 3. In this case, operation of the device 1 is not impaired. This means that the logged voltage profile can be deleted. As an alternative, the logged voltage profile can be saved for information purposes in this case.

A drop of the battery voltage Vbat, and therefore of the voltage VDD, can also occur if large currents are drawn from the battery 2 and the battery 2 is weak. This might be the case if the pump is activated while the background light of a display is on. However, the background light and the pump are typically activated for several seconds, such that the time span between the beginning of the logging, or the last logged value before VDD has fallen to the reset voltage level, and the power-up entry is quite long (i.e. in the range of several seconds), and the battery voltage Vbat does not drop as deep as to zero or near zero. Therefore, power interruption caused by large power consumption in combination with a weak battery can also be identified.

## Claims

1. Medical infusion pump (1), comprising a power source (2) and a processor (3), **characterized by** a voltage supervisor (4), wherein the voltage supervisor (4) is configured to monitor the input voltage (VDD) of the processor (3), the voltage supervisor (4) is configured to respond if the voltage (VDD) drops to a predetermined level which is above a reset voltage level of the processor (3), and below a minimum voltage level which may occur during normal operation of the medical infusion pump (1), and the processor (3) is configured to start logging the voltage (Vbat) of the power source (2) when the voltage supervisor (4) responds.

2. Medical infusion pump (1) according to claim 1, wherein the processor (3) is configured to determine the cause of an interruption of the power supply to the processor (3) by comparing the logged power source voltage to predetermined reference power source voltage profiles.

3. Medical infusion pump (1) according to one of claims 1 to 2, wherein the power source (2) is a single-use battery or a rechargeable battery.

4. Medical infusion pump (1) according to one of claims 1 to 3, **characterized by** a voltage converter (6) between the power source (2) and the processor (3).

5. Medical infusion pump (1) according to one of claims 1 to 4, wherein the voltage supervisor (4) is connected to an interrupt input port of the processor (3).

6. Medical infusion pump (1) according to one of claims 1 to 5, wherein the processor (3) is configured to log the power source voltage (Vbat) until the processor (3) is reset.

7. Medical infusion pump (1) according to one of claims 1 to 5, wherein the processor (3) is configured to log the power source voltage (Vbat) until the voltage supervisor (4) stops responding.

8. Medical infusion pump (1) according to one of claims 1 to 7, wherein the processor (3) is configured to perform alternative logging of power source voltage values to different memories.

9. Medical infusion pump (1) according to one of claims 1 to 8, wherein the processor (3) is configured to log a timestamp together with the power source voltage (Vbat) values.

10. Method for determining the cause of an interruption of a power supply provided by a power source (2) in a medical infusion pump (1), the medical device comprising a processor (3), **characterized by** the steps of detecting if the input voltage (VDD) of the processor (3) drops to a predetermined level which is higher than a reset voltage level of the processor (3), and below a minimum voltage level which may occur during normal operation of the medical infusion pump (1), starting to log the voltage (Vbat) of the power source (2) when the input voltage (VDD) has dropped to the predetermined voltage level and determining the cause of an interruption of the power supply from the logged power source voltage (Vbat).

11. Method according to claim 10, wherein the power source voltage (Vbat) is logged until the processor input voltage (VDD) drops to the reset voltage level of the processor (3).

12. Method according to claim 10, wherein the power source voltage (Vbat) is logged until the processor input voltage (VDD) rises to or above the predetermined voltage.

13. Method according to one of claims 10 to 12, wherein the power source voltage (Vbat) values are logged alternatively to different memories.

14. Method according to one of claims 10 to 13, wherein a timestamp is logged together with the power source voltage (Vbat).

## Patentansprüche

1. Medizinische Infusionspumpe (1), aufweisend eine Energiequelle (2) und einen Prozessor (3), **gekennzeichnet durch** eine Spannungsüberwachung (4), wobei die Spannungsüberwachung (4) dazu eingerichtet ist, die Eingangsspannung (VDD) des Prozessors (3) zu überwachen, die Spannungsüberwachung (4) dazu eingerichtet ist, anzusprechen wenn die Spannung (VDD) auf ein vorbestimmtes Level fällt, das über einem Resetspannungslevel des Prozessors (3) und unter einem Mindestspannungslevel, das während dem normalen Betrieb der medizinischen Infusionspumpe (1) auftreten kann, liegt, und der Prozessor (3) dazu eingerichtet ist, das Protokollieren der Spannung (Vbat) der Energiequelle (2) zu beginnen, wenn die Spannungsüberwachung (4) anspricht.

2. Medizinische Infusionspumpe (1) nach Anspruch 1, wobei der Prozessor (3) dazu eingerichtet ist, die Ursache einer Unterbrechung der Spannungsversorgung des Prozessors (3) durch Vergleichen der protokollierten Energiequellenspannung mit vorbestimmten Referenz-Energiequellen-Spannungsprofilen zu bestimmen.

3. Medizinische Infusionspumpe (1) nach einem der Ansprüche 1 bis 2, wobei die Energiequelle (2) eine Einmal-Batterie oder eine wiederaufladbare Batterie ist.

4. Medizinische Infusionspumpe (1) nach einem der Ansprüche 1 bis 3, **gekennzeichnet durch** einen Spannungswandler (6) zwischen der Energiequelle (2) und dem Prozessor (3).

5. Medizinische Infusionspumpe (1) nach einem der Ansprüche 1 bis 4, wobei die Spannungsüberwachung (4) mit einem Interrupt-Eingang des Prozessors (3) verbunden ist.

6. Medizinische Infusionspumpe (1) nach einem der Ansprüche 1 bis 5, wobei der Prozessor (3) dazu eingerichtet ist, die Energiequellenspannung (Vbat) zu protokollieren, bis der Prozessor (3) zurückgesetzt wird.

7. Medizinische Infusionspumpe (1) nach einem der Ansprüche 1 bis 5, wobei der Prozessor (3) dazu eingerichtet ist, die Energiequellenspannung (Vbat) zu protokollieren, bis dass die Spannungsüberwachung (4) aufhört ansprechen.

8. Medizinische Infusionspumpe (1) nach einem der Ansprüche 1 bis 7, wobei der Prozessor (3) dazu eingerichtet ist, alternatives Protokollieren der Energiequellen-Spannungswerte in verschiedene Speicher durchzuführen.

9. Medizinische Infusionspumpe (1) nach einem der Ansprüche 1 bis 8, wobei der Prozessor (3) dazu eingerichtet ist, einen Zeitstempel zusammen mit den Werten der Energiequellenspannung (Vbat) zu protokollieren.

10. Verfahren zur Bestimmung der Ursache einer Unterbrechung einer Energieversorgung, die von einer Energiequelle (2) erbracht wird, in einer medizinischen Infusionspumpe (1), wobei das medizinische Gerät einen Prozessor (3) aufweist, **gekennzeichnet durch** die Schritte des Detektierens, ob die Eingangsspannung (VDD) des Prozessors (3) auf ein vorbestimmtes Level fällt, das höher ist als ein Resetspannungslevel des Prozessors (3) und niedriger als ein Mindestspannungslevel, das während des normalen Betriebs der medizinischen Infusionspumpe (1) auftreten kann, des Startens des Protokollierens der Spannung (Vbat) der Energiequelle (2) wenn die Eingangsspannung (VDD) auf das vorherbestimmte Spannungslevel gefallen ist, und des Bestimmens der Ursache einer Unterbrechung der Energieversorgung aus der protokollierten Energiequellenspannung (Vbat).

11. Verfahren nach Anspruch 10, wobei die Energiequellenspannung (Vbat) protokolliert wird, bis das die Prozessoreingangsspannung (VDD) auf das Resetspannungslevel des Prozessors (3) fällt.

12. Verfahren nach Anspruch 10, wobei die Energiequellenspannung (Vbat) protokolliert wird, bis dass die Prozessoreingangsspannung (VDD) auf oder über die vorbestimmte Spannung steigt.

13. Verfahren nach einem der Ansprüche 10 bis 12, wobei die Werte der Energiequellenspannung (Vbat) alternativ in verschiedene Speicher protokolliert werden.

14. Verfahren nach einem der Ansprüche 10 bis 13, wobei ein Zeitstempel zusammen mit der Energiequellenspannung (Vbat) protokolliert wird.

## Revendications

1. Pompe à perfusion médicale (1), comprenant une source d'énergie (2) et un processeur (3), **caractérisée par** un superviseur de tension (4), dans laquelle le superviseur de tension (4) est configuré pour surveiller la tension d'entrée (VDD) du processeur (3), le superviseur de tension (4) étant configuré pour répondre si la tension (VDD) chute à un niveau prédéterminé qui est supérieur à un niveau de tension de réinitialisation du processeur (3) et inférieur à un niveau de tension minimal qui peut survenir pendant le fonctionnement normal de la pompe à perfusion médicale (1), et le processeur (3) est configuré pour commencer à enregistrer la tension (Vbat) de la source d'énergie (2) lorsque le superviseur de tension (4) répond.

2. Pompe à perfusion médicale (1) selon la revendication 1, dans laquelle le processeur (3) est configuré pour déterminer la cause d'une interruption de la source d'énergie au processeur (3) en comparant la tension de la source d'énergie enregistrée à des profils de tension de référence prédéterminés de la source d'énergie.

3. Pompe à perfusion médicale (1) selon l'une des revendications 1 à 2, dans laquelle la source d'énergie (2) est une batterie à usage unique ou une batterie rechargeable.

4. Pompe à perfusion médicale (1) selon l'une des revendications 1 à 3, **caractérisée par** un convertisseur de tension (6) entre la source d'énergie (2) et le processeur (3).

5. Pompe à perfusion médicale (1) selon l'une des revendications 1 à 4, dans laquelle le superviseur de tension (4) est relié à un orifice d'entrée d'interruption du processeur (3).

6. Pompe à perfusion médicale (1) selon l'une des revendications 1 à 5, dans laquelle le processeur (3) est configuré pour enregistrer la tension de la source d'énergie (Vbat) jusqu'à ce que le processeur (3) soit réinitialisé.

7. Pompe à perfusion médicale (1) selon l'une des revendications 1 à 5, dans laquelle le processeur (3) est configuré pour enregistrer la tension de la source d'énergie (Vbat) jusqu'à ce que le superviseur de tension (4) arrête de répondre.

8. Pompe à perfusion médicale (1) selon l'une des revendications 1 à 7, dans laquelle le processeur (3) est configuré pour réaliser un enregistrement alternatif de valeurs de tension de la source d'énergie dans différentes mémoires.

9. Pompe à perfusion médicale (1) selon l'une des revendications 1 à 8, dans laquelle le processeur (3) est configuré pour enregistrer un horodatage conjointement aux valeurs de tension de la source d'énergie (Vbat).

10. Procédé de détermination de la cause d'une interruption d'un apport d'énergie fourni par une source d'énergie (2) dans une pompe à perfusion médicale (1), le dispositif médical comprenant un processeur (3), **caractérisé par** les étapes consistant à détecter si la tension d'entrée (VDD) du processeur (3) chute à un niveau prédéterminé qui est supérieur à un niveau de tension de réinitialisation du processeur (3) et inférieur à un niveau de tension minimal qui peut survenir pendant le fonctionnement normal de la pompe à perfusion médicale (1), à commencer à enregistrer la tension (Vbat) de la source d'énergie (2) lorsque la tension d'entrée (VDD) a chuté au niveau de tension prédéterminé et à déterminer la cause d'une interruption de l'apport d'énergie à partir de la tension de la source d'énergie enregistrée (Vbat).

11. Procédé selon la revendication 10, dans lequel la tension de la source d'énergie (Vbat) est enregistrée jusqu'à ce que la tension d'entrée du processeur (VDD) chute au niveau de tension de réinitialisation du processeur (3).

12. Procédé selon la revendication 10, dans lequel la tension de la source d'énergie (Vbat) est enregistrée jusqu'à ce que la tension d'entrée du processeur (VDD) augmente à la tension prédéterminée ou au-delà.

13. Procédé selon l'une des revendications 10 à 12, dans lequel les valeurs de tension de la source d'énergie (Vbat) sont enregistrées alternativement dans différentes mémoires.

14. Procédé selon l'une des revendications 10 à 13, dans lequel un horodatage est enregistré conjointement à la tension de la source d'énergie (Vbat).
